# EUROPEAN PATENT SPECIFICATION

(11) **EP 2 231 044 B1**
(45) Date of publication and mention of the grant of the patent: **07.03.2012**
(21) Application number: 09701633.1
(22) Date of filing: 14.01.2009
(51) Int. Cl.: A61B 17/80, A61B 17/88, A61B 17/70, A61B 17/064, A61B 17/068

(54) **SPINAL OSTEOSYNTHESIS DEVICE**
WIRBELSÄULEN-OSTEOSYNTHESEVORRICHTUNG
DISPOSITIF D'OSTÉOSYNTHÈSE RACHIDIENNE

(30) Priority: 17.01.2008 FR 0850285
(43) Date of publication of application: 29.09.2010
(73) Proprietor: Warsaw Orthopedic, Inc., Warsaw, IN 46581 (US)
(72) Inventor: DUPONT, Phillipe, F-77410 Claye Souilly (FR); ASSAKER, Richard, B-7540 Kain (BE); D'AMORE, Jean-francois, F-62780 Stella Plage (FR); LEMAITRE, Philippe, F-01170 Crozet (FR); MANGIONE, Paolo Fernando Dr., F-33600 Pessac (FR)
(74) Representative: Viering, Jentschura & Partner
(86) International application number: PCT/US2009/030904
(87) International publication number: WO 2009/091770

(56) References cited:
- DE-U1- 20 001 879
- US-A- 5 785 713
- US-A1- 2005 043 732
- US-A1- 2006 241 615
- US-A1- 2007 270 965
- US-B1- 6 187 009
- US-B1- 6 602 255
- US-B1- 6 602 255
- US-B2- 6 695 845
- US-B2- 7 115 129
- US-B2- 7 115 129
- US-B2- 7 309 340
- US-B2- 7 309 340

## Description

The invention relates to orthopaedic surgery and, more precisely, spinal osteosynthesis devices made up of plates designed to be implanted on the spine to stabilise the relative positions of two or more consecutive vertebrae.

In the case in which a patient suffers from a pathology that deteriorates the functionality of one or more intervertebral disks, recourse is often made to the installation of a metallic plate, fixed by screws to the vertebrae surrounding the afflicted disk(s). It has the function of stabilising the relative positions of the vertebrae involved by binding them rigidly. These plates are used in particular in the cervical region and are installed by the anterior or posterior pathway. It is important that these plates be solidly anchored to the vertebrae and cannot become progressively disengaged from them, whence the customary use of screws for their fixation.

The screws that fix these plates to the vertebrae are independent parts, which must therefore be manufactured separately from the plates. It is also necessary to provide the plates with anti-withdrawal devices so as to prevent the screws from disengaging from their housing, for example disks partially covering the heads of the screws. The necessity of implanting the screws and the anti-withdrawal plates is also a source of prolongation of the duration of installation.

US 2007/0270965 A1 discloses a spinal plate positioning system including a rigid or flexible spinal plate concave along its longitudinal direction, centering member and implant.

US 2006/0241615 A1 discloses a plating system for stabilization of a bony segment, including a plate engageable to at least first and second bony elements, such as vertebrae, via fasteners, such as bone screws.

US 6187 009 B1 discloses an implant including a base body in the form of a plate having upper and lower sides, the lower side having extensions for insertion in the bone.

DE 200 01 879 U1 discloses a bone plate for connecting two vertebrae, comprising a plate shaped connection section for each vertebrae, and at least one through hole for a bone screw in each connection section.

US 5 785 713 A discloses a bone fixation apparatus including a body portion and plurality of leg portions extending from a lower surface of the body portion. The body is resiliently deformable between an initial orientation and an insertion orientation upon application of a force to the body portion. The leg portions are oriented at an angle relative to the opposite leg portion when the body is in the initial orientation, wherein, to facilitate deformation of the plate to bring the extensions into a substantially parallel orientation, the upper surface of the body portion may be concave in case the leg portions initially extend toward each other or the lower surface of the body portion may be concave in case the leg portions initially extend away from each other.

US 7 309 340 B1 discloses a plating system comprising a bone plate having a longitudinal and transversal curvature, and comprising bone fasteners for securing the bone plate.

US 7 115 129 B2 discloses a bone compression device comprising a plate having a pre-formed shape, a deformed shape, and at least one elastic shape therebetween.

US 6 602 255 B1 discloses a spinal plate having a curvatures in longitudinal and transversal directions, openings, and bone-anchoring members to be inserted into the openings.

US 2004/0127902 A1 discloses a vertebra body plate comprised of a plate main body formed in a slightly curved manner and provided with spikes thereon.

The objective of the invention is to propose such a plate with a new design that makes it possible to dispense with the anti-withdrawal devices that are usually associated with the screws, and also with the screws themselves.

To this end, the invention provides a device according to claim 1. Further embodiments of the invention are described in the dependent claims.

The invention has for an object a spinal osteosynthesis device made up of a plate designed to be fixed by anchoring means overlapping at least two vertebrae of a patient, wherein said plate presents at rest a curvature in its longitudinal direction, and/or its transverse direction, and in that it presents properties of elasticity that make possible straightening of said curvature at the time of installation of the plate.

The plate may present a curvature in both its longitudinal and transverse directions.

Said anchoring means are anchoring studs made solidly affixed to the plate.

The lateral edges of the plate may present at least one concavity.

The anchoring studs may present anti-return edges or anti-return barbs.

The plate may be designed to be placed to overlap two vertebrae and comprise four anchoring studs, two of said studs being designed to penetrate into one of the vertebrae and the other two of said studs being designed to penetrate into the other vertebra.

The plate may be designed to be placed to overlap three vertebrae and comprise five anchoring studs with one designed to penetrate into the central vertebra.

The plate may comprise depressions or orifices wherein a bone graft placed between the vertebrae and compressed by the action of the plate may penetrate.

As already understood, the invention rests on the use of a plate which, at rest, is curved in at least one of its longitudinal and transverse directions, and is capable of being elastically straightened out at the time of its installation, for example by means of an instrument specially adapted for this purpose which will also be described. Preferably, the plate is curved in both its longitudinal and transverse directions. It is, preferably, fitted with means for anchoring to the vertebrae solidly affixed to it and making possible an easy installation and a reliable bond between the corresponding vertebra and the plate. Anchoring studs of circular or flattened cross-section, capable of comprising anti-return edges or barbs, are particularly indicated for this purpose. The surgeon penetrates these studs, by impact on the straightened plate, into orifices made beforehand in the vertebrae. The tendency of the plate to regain its initial curved shape after its installation causes the studs to exert a push on the walls of their orifices, which prevents their spontaneous extraction. In the case in which the plate is, at rest, curved in its longitudinal direction, a tendency is also observed for coming together or for separation of the vertebrae in the region of installation (on the one hand according as the longitudinal curvature of the plate in its normal state tends to make the studs convergent or divergent, and on the other hand according to anterior or posterior positioning of the plate on the spine), which the surgeon may also take advantage of to accelerate bone fusion by compression of a graft, or to locally straighten the curvature of the spine.

The invention will be better understood upon reading of the following description, given with reference to the following appended figures:
- Figure 1 which shows, in a profile view, an example of a plate according to the invention;
- Figure 2 which shows this same plate in a perspective view;
- Figure 3 which shows this same plate in a top view;
- Figure 4 which shows, in a perspective view, another example of a plate according to the invention;
- Figures 5 and 6 which show, in perspective, both surfaces of another example of a plate according to the invention;
- Figure 7 which shows, in a perspective view, an instrument for the installation of a plate according to the invention;
- Figure 8 which shows, in a perspective view, a detail of this instrument with a plate placed between its jaws;
- Figure 9 which shows the instrument and the plate seen in profile in their position of Figure 8;
- Figure 10 which shows the instrument and the plate seen in profile in their position in which the plate is ready to be installed.

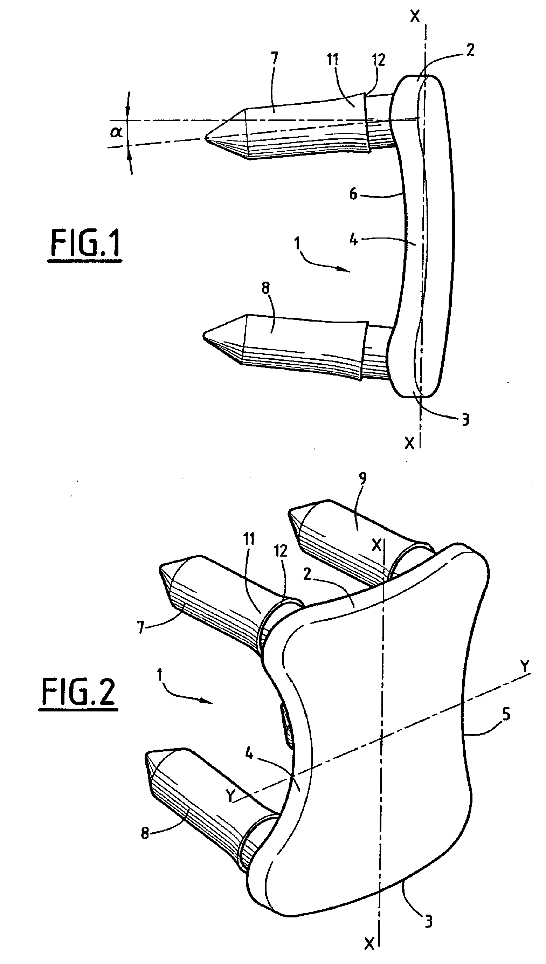

The example of plate 1 according to the invention shown in Figures 1 and 2 is designed to be installed overlapping two consecutive vertebrae. Plate 1 comprises an upper edge 2, a lower edge 3 and two lateral edges 4, 5. Lateral edges 4, 5 present a concavity, in such a way that plate 1 is less wide at its median part than at its upper and lower parts, which promotes its elastic deformation. Upper 2 and lower 3 edges preferably present, as shown, a curvature which confers on plate 1 a curved shape in its transverse direction (YY) enabling the rear surface 6 of plate 1 to roughly fit the shape of the vertebrae on which it is installed when this installation takes place by the anterior pathway.

According to the invention, plate 1 presents a curvature in its longitudinal (XX) direction which, at rest, confers a curved shape on plate 1.

Also according to the invention, plate 1 may be elastically deformed by means of an appropriate instrument, in such a way as to reduce, even eliminate, this curvature at the time of installation of the plate, as will be explained later on.

In the example shown, plate 1 also comprises a curvature in its transverse direction (YY), a curvature that may also be reduced or eliminated at the time of elastic deformation of the plate at the time of its installation.

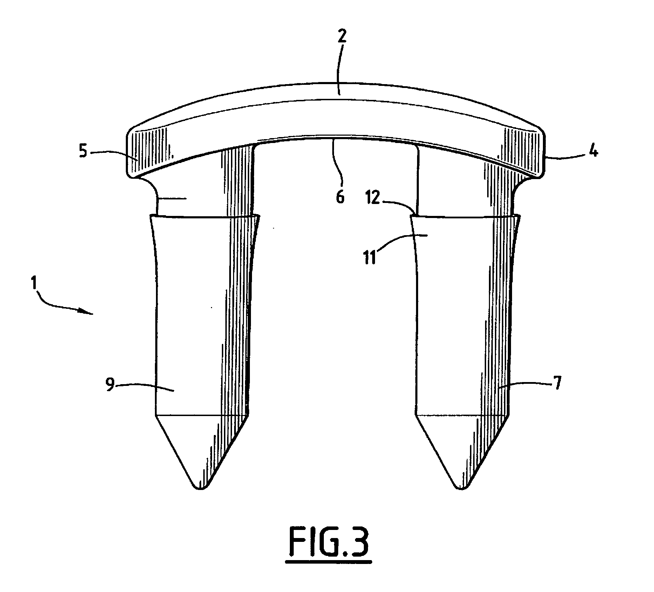

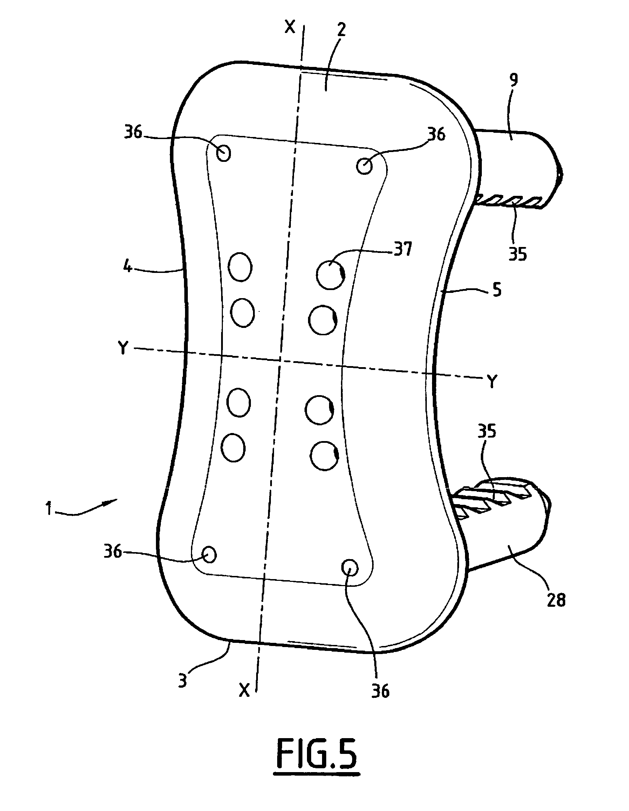

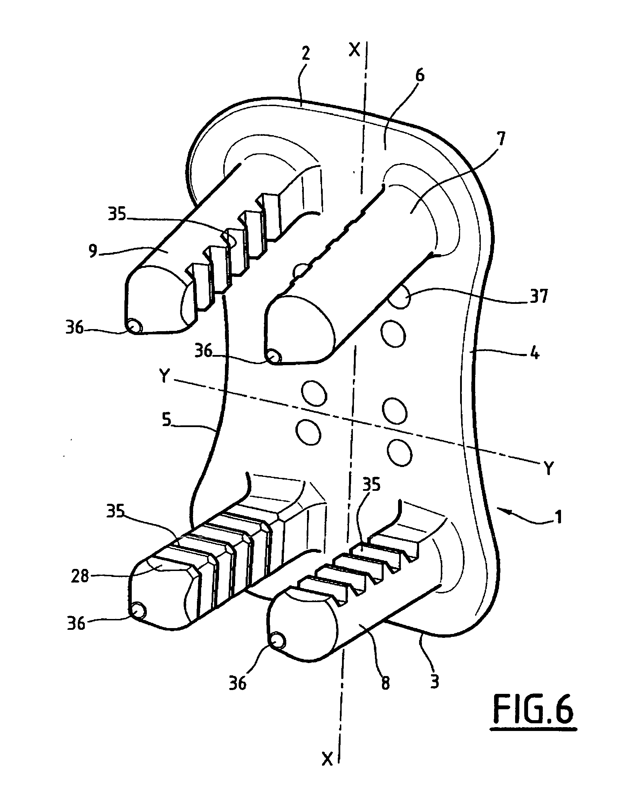

On its rear surface 6, plate 1 comprises anchoring studs 7, 8, 9, 28 numbering four in the example shown (three are visible in Figures 1 to 3). These studs 7, 8, 9, 28 are made of the same piece with plate 1: it is therefore not necessary to provide separate parts such as screws for the fixation of plate 1 to the vertebrae, which simplifies the installation of plate 1 and the management of the stock of parts. Studs 7, 8, 9, 28 have, in the example shown in Figures 1, 2, 3, 8, 9, 10, a roughly circular cross-section. It could be flattened to optimise support on spongy bone, as is stud 28 in the alternative shown in Figures 5 and 6. In the example shown in Figures 1, 2, 3, 8, 9, 10, studs 7, 8, 9, 28 each comprise a localised bulge 11 presenting an edge 12 in the proximity of rear surface 6 of plate 1. This edge plays the role of an anti-return barb and tends to prevent spontaneous extraction of the plate after its positioning. As an alternative, one or more true barbs could be provided for on studs 7, 8, 9, 28 to make up this anti-return device. Such barbs 35 are shown in the alternative to Figures 5 and 6. It is self-evident that all studs 7, 8, 9, 28 are not necessarily identical. This is the case in the alternative in Figures 5 and 6, in which stud 28 is of approximately square cross-section, except at its distal end ensuring the penetration of stud 28 into the vertebra.

Plate 1 is, preferably, made of carbon or of a biocompatible polymer presenting good mechanical properties such as PEEK (polyether-ether-ketone), materials that have the advantage of being radio-transparent. This radio-transparency makes it possible to be aware of the actual character of the bone fusion with a simple X-ray. A biocompatible metal (titanium, stainless steel, form-memorising alloy of super-elastic form, etc.) would also be useful if the absence of radio-transparency is accepted by the surgeon. The alternative to Figures 5 and 6 locally presents non-radio-transparent markers 36 on the external surface of plate 1 and at the ends of studs 7, 8, 9, 28, in such a way that the principal points of plate 8 would be detectable with an X-ray, even if plate 1 is made of a radio-transparent material.

The various dimensions of plate 1 are chosen as a function of the size of the vertebrae involved, the future positioning of plate 1 and the mechanical properties of the material. It is necessary for plate 1 to present sufficient elasticity so that its curvature in one at least of the longitudinal (XX) and transverse (YY) directions can be greatly reduced, even eliminated, during installation. In this way, after its installation plate 1 will tend to regain its initial shape in such a way that studs 7, 8, 9, 28 will exert an increased force on the walls of their respective orifices with respect to what would take place if plate 1 were not elastically deformable at the time of its installation. Thus, the resistance of plate 1 to its extraction is increased.

Studs 7, 8, 9, 28 are not, preferably, parallel at rest, but present a convergence that makes the extraction of plate 1 after its installation even more difficult. This convergence is expressed by an angle α between the axes of studs 7, 8, 9, 28 and the perpendicular to rear surface 6 at the starting point of the corresponding stud 7, 8, 9, 28.

Moreover, in the example shown in which a curvature of plate 1 is constructed in its longitudinal (XX) direction, the tendency for the return of plate 1 to its initial shape has the effect of tending to bring together the vertebrae on which plate 1 is installed. Permanent constraint of the bone graft which was inserted beforehand between the vertebrae to replace a diseased disk is achieved in this way. The fusion of the vertebrae is accelerated on this account. According to whether positioning of plate 1 is anterior or posterior, and also according to the depth of penetration of studs 7, 8, 9, 28 into the vertebrae, the bringing together of the vertebrae can be carried out in their anterior region or in their posterior region, or in their median region to bring them together in a direction roughly parallel to the orientation of the spine in the area under consideration. In the cervical area, where the invention finds an application example that is privileged but not exclusive, it will be generally be favourable to provide for an installation of the plate by the anterior pathway, and for studs 7, 8, 9, 28 that are sufficiently long so that bringing together of the vertebrae takes place in their posterior part. In this way, the natural curvature of the spine in the area under consideration is restored.

The installation of plate 1 takes place as follows.

The surgeon contrives, in the vertebrae involved, orifices that will make it possible to initiate the insertion of anchoring studs 7, 8, 9, 28. The center distances of the axes of the orifices correspond to the distances between studs 7, 8, 9, 28 when plate 1 is in the straightened state.

Then, if it has not been done previously, he inserts between the vertebrae the bone graft that will make it possible to achieve fusion of the vertebral end-plates. This bone graft can be inserted alone, or, as is well-known, be confined in an intervertebral cage which ensures its maintenance in place and contributes to establishing the intervertebral distance after the bone fusion.

Then, by means of an instrument such as that which will be described later, the surgeon grasps plate 1 and exerts on it a force which reduces or eliminates its longitudinal curvature along (XX), so as to confer on the ends of studs 7, 8, 9, 28 center distances of the axes corresponding to those of the orifices. In this position, studs 7, 8, 9, 28 are preferably found with roughly parallel orientations so as to facilitate their introduction.

Then the surgeon places anchoring studs 7, 8, 9, 28 in the orifices of the vertebrae, and impacts plate 1, for example by striking the instrument with a hammer which straightens the plate it and maintains it, in such a way as to push anchoring studs 7, 8, 9, 28 down into their orifices, which they enlarge themselves so as to be blocked in them. The rear surface 6 of plate 1 thus comes in contact with the vertebrae.

Then the straightening force on plate 1 is broken off, for example by disengaging the instrument from plate 1. The latter therefore tends to regain its initial curved shape, which makes extraction of plate 1 more difficult for the reasons that were mentioned. Also, plate 1 exerts a force to bring the vertebrae together and compresses the bone graft. Thus there is an increase in the possibilities and speed of fusion of the vertebral end-plates compared to what would take place in the absence of compression. The localisation and intensity of this compression are controlled by the choice of material and dimensions of the plate.

As in the example shown in Figures 5 and 6, it is possible to provide on plate 1, whatever its configuration might be in other respects, depressions or orifices 37 wherein bone graft can penetrate at the time of its compression. By solidifying, this graft contributes to maintaining plate 1 in place. Preferably, as shown, these orifices cross plate 1.

By way of non-restrictive example, for a plate made of PEEK designed to be implanted by the posterior pathway overlapping two cervical vertebrae, of length 22 mm, of maximum width 16 mm and of thickness 1.8 mm, it is possible to confer on the longitudinal curvature a radius of 170 mm and on the lateral concavities a radius of 14.5 mm. The anchoring studs 7, 8, 9 have a length of 13 mm and a diameter of 3.5 mm, which goes to 4 mm at edge 12. α is 6o.

It is also possible to confer on plate 1 a longitudinal curvature opposite to that which has been described and shown, so that, after implantation, anchoring studs 7, 8, 9, 28 tend to separate from each other instead of coming together. In this way plate 1 may be used to locally straighten a curvature of the vertebral column, if it is implanted, for example, in the anterior thoracic region or in the posterior lumbar region.

When plate 1 extends overlapping two consecutive vertebrae, the presence of four anchoring studs 7, 8, 9, 28 is generally advised. Only two of them could be provided, in particular if plate 1 is designed to be implanted on the smallest cervical vertebrae. It is also possible to provide a higher number of studs 7, 8, 9, 28 for plates 1 designed to be implanted on the largest vertebrae of the spine.

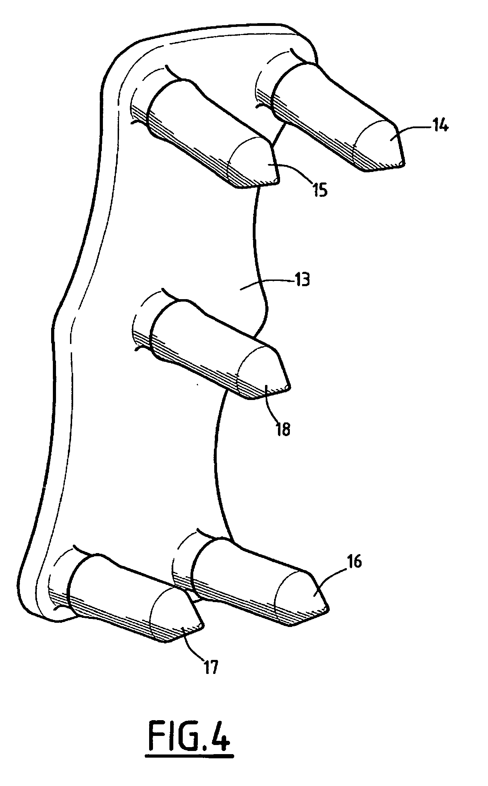

When plate 1 extends over three consecutive vertebrae, it is possible to use a plate 13 as shown in Figure 4, fitted with two studs 14, 15 to be hammered into the upper vertebra, two studs 16, 17 to be hammered into the lower vertebra, and a single stud 18 to be hammered into the central vertebra. Plate 13, from the point of view of its outer shape, appears roughly as the junction of two plates 1 of Figures 1-3 of which the upper edge of the one is merged with the lower edge of the other. The anchoring 18 on the central vertebra constitutes a neutral point with respect to which the two halves of plate 13 articulate at the time of the deformations of plate 13.

It would also be conceivable to not integrate the anchoring elements with plate 1, 13, but to carry out this anchoring by means, for example, of screws crossing orifices worked into plates 1, 13, on the condition of ensuring that the forces exerted by the plate to tend to return to its initial curved configuration would be properly transmitted to the anchoring elements by means of a sufficient contact surface with the screws, in particular with their threaded rods. The tendency for return of plate 1 to the curved configuration makes it possible to increase the friction between the rods of the screws and the walls of their orifices, which makes withdrawal of the screws more difficult and makes it possible to eliminate the anti-withdrawal devices which are ordinarily associated with them.

It must be understood that the configurations shown in Figures 1 to 6 are in no way restrictive of the invention. In particular, it is possible to combine various characteristics each present in only one of the alternatives shown, if they are not manifestly incompatible.

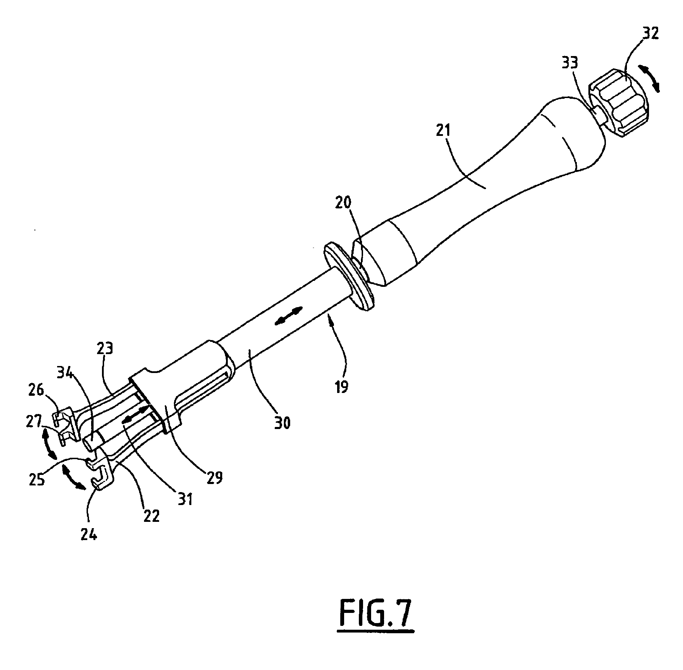

Description will now be made of an example of an instrument specially designed for the manipulation and positioning of plates 1, 13 according to the invention. It is shown in Figures 7 to 10.

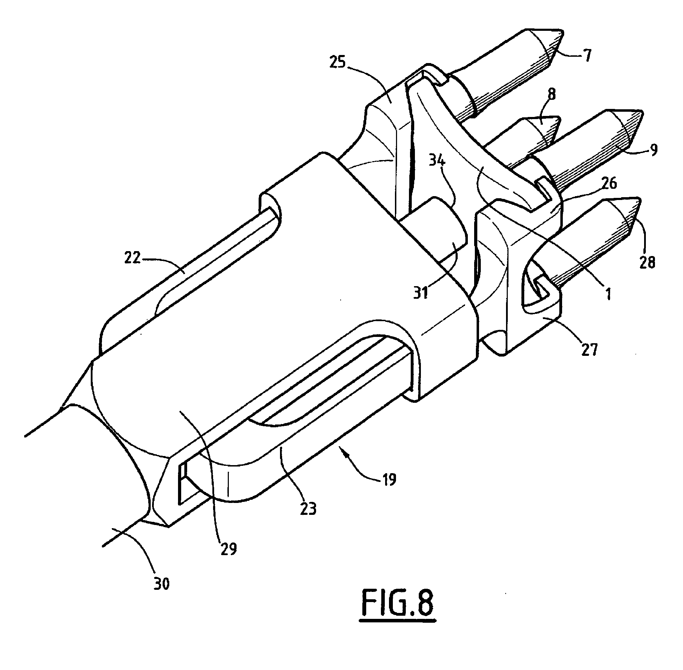

Instrument 19 comprises a tubular rod 20 fitted with a handle 21 making it possible for the surgeon to hold and manipulate the instrument. At the front end of rod 20 there are two jaws 22, 23 capable of turning in the same plane around two parallel axes (not visible in the figures). They are each fitted at their free end with claws 24, 25, 26, 27 enabling the prehension of plate 1 by the base of anchoring studs 7, 8, 9, 28 and leaving it free to be straightened. A slide 29 surrounds jaws 22, 23 at their rear sections. The advance of slide 29 is controlled by a sleeve 30 which is solidly affixed to it and surrounds rod 20. When the surgeon moves sleeve 30 so as to progress it toward the front of instrument 19, slide 29 is carried along by sleeve 30 and tends to bring jaws 22, 23 close to each other. If a plate 1 was placed between claws 24, 25, 26, 27, the latter come together, so as to achieve a clamping of plate 1 which is thus maintained firmly between the claws, as shown in Figure 8.

Instrument 19 also comprises, inserted in tubular rod 20, a straightening rod 31 that can progress into tubular rod 20 by means of threads worked into its surface, when the surgeon turns an adjusting wheel 32 placed at rear end 33 of straightening rod 31.

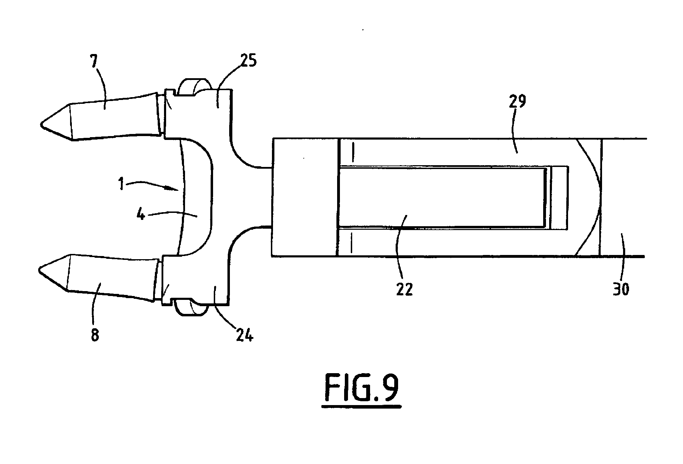

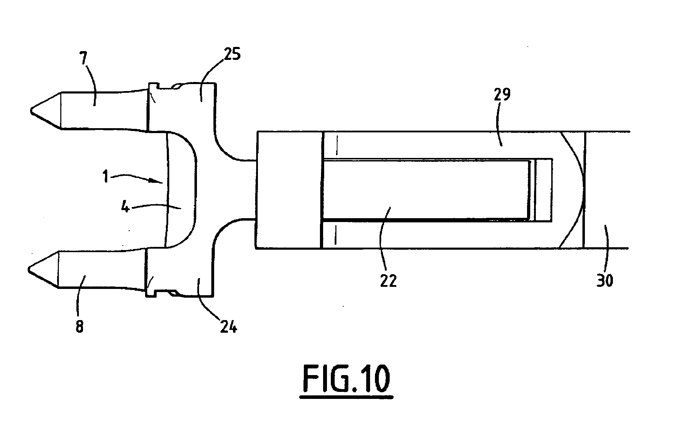

When plate 1 has been placed and clamped between claws 24, 25, 26, 27 of jaws 22, 23, the surgeon turns adjusting wheel 32 in such a way as to bring front end 34 of straightening rod 31 in contact with plate 1. Instrument 19 is then in the position of Figures 8 and 9 in which plate 1 is maintained in the curved state. Then the surgeon continues to turn adjusting wheel 32 so as to exert a push on plate 1 through straightening rod 31 which continues to advance. Under the effect of this pushing, plate 1 is elastically deformed and ends up in a state in which it no longer appreciably presents a curvature and in which anchoring studs 7, 8, 9, 28 are, preferably, roughly parallel, as shown in Figure 10. At this time, plate 1 is ready to be positioned. The surgeon then brings it close to the patient's vertebrae, inserts anchoring studs 7, 8, 9, 28 into the orifices made beforehand in the vertebrae, and impacts the rear end of instrument 19 by means of a hammer to make the studs 7, 8, 9, 28 penetrate deeply into the vertebrae. Once penetration has been achieved, the surgeon turns adjusting wheel 32 to carry out the withdrawal of the straightening rod, and then turns sleeve 30 to carry out the unclamping of jaws 22, 23. Plate 1 is thus released from instrument 19 and can play its role in the stabilisation of the vertebrae on which it is implanted.

Instrument 19 may also be adapted to the case in which plate 1 presents, at rest, a curvature opposite to that shown in the figures, as envisaged above. In this case, straightening is obtained by making front end 34 of straightening rod 31 solidly fixed to plate 1 (for example, by means of a threading or a bayonet fitting), and by progressing straightening rod 31 toward the rear of instrument 19 in such a way as to cause it to exert a traction on plate 1. If provision is made for the possibility of changing the part of end 34 of the straightening rod as a function of the configuration of plate 1, the same instrument 19 can be used for both uses. It is also possible to provide for the possibility of changing jaws 22, 23 as a function of the configuration and dimensions of plate 1 that is to be implanted. In particular, these jaws can, in a way obvious to those skilled in the art, be adapted to the grasping of plates 13 that are capable of extending over more than two vertebrae.

Modifications can be provided to the instrument, while preserving the essential functions. For example, jaws 22, 23 and/or claws 24, 25, 26, 27 could be replaced by any other means of prehension that are appropriate to and compatible with plates 1, 13 used, and slide 29 could be replaced by any other means for bringing jaws 22, 23 together.

## Claims

1. Spinal osteosynthesis device consisting of a plate (1, 13) provided with anchoring means and intended to be affixed, by said anchoring means, straddling over at least two vertebrae of a patient, wherein said plate (1,13) has, when unstressed, a curvature along both its longitudinal (XX) and transversal (YY) directions
and wherein the plate (1,13) possesses an elasticity which allows said curvature to be elastically straightened during the setting of the plate (1, 13) so that the plate (1, 13) tends to regain its initial curved shape, and
wherein said anchoring means are anchoring studs (7, 8, 9, 28; 14, 15, 16, 17, 18) made solidly affixed to the plate (1, 13) so that the tendency of the plate (1, 13) to regain its initial curved shape after its installation causes the studs to exert a push on the walls of their orifices in the vertebrae.

2. Device according to claim 1, **characterized in that** the plate (1, 13) has, when unstressed, a curvature along its transversal direction (YY) that produces a concavity of the rear surface 6 of the plate (1, 13) along its transversal direction (YY).

3. Device according to one of claims 1 to 2, **characterized in that** said anchoring studs (7, 8, 9, 28; 14, 15, 16. 17, 18) made integral with the plate (1, 13).

4. Device according one of claims 1 to 3, **characterized in that** the lateral edges (4, 5) of the plate (1, 13) have at least one concavity.

5. Device according claim 3 or 4, **characterized in that** the anchoring studs (7, 8, 9, 28; 14, 15, 16, 17, 18) have anti-return ridges (12) or anti-return barbs (35).

6. Device according to one of claims 3 to 5, **characterized in that** the plate (1) is intended to straddle over two vertebrae and has four anchoring studs (7, 8, 9, 28), two (7, 9) of which being intended to penetrate into one of said vertebrae and the other two (8, 28) studs being intended to penetrate into the other of said vertebrae.

7. Device according to one of claims 3 to 5, **characterized in that** the plate (13) is intended to straddle over three vertebrae and has five anchoring studs (14, 15, 16, 17, 18), one (18) of them being intended to penetrate into the central vertebra.

8. Device according to one of claims 1 to 7, **characterized in that** the plate (1, 13) has depressions or orifices (37) into which a bone graft placed between the vertebrae and compressed by the action of the plate (1, 13) can penetrate.

## Patentansprüche

1. Wirbelsäulen-Osteosynthesevorrichtung, bestehend aus einer Platte (1, 13), die mit Verankerungsmitteln versehen ist und vorgesehen ist, um durch die Verankerungsmittel fixiert zu sein, die mindestens zwei Wirbel eines Patienten überspannt, wobei die Platte (1, 13), wenn sie unbelastet ist, entlang sowohl ihrer Längsrichtung (XX) als auch ihrer Querrichtung (XX) eine Krümmung aufweist, und wobei die Platte (1, 13) eine Elastizität aufweist, die ermöglicht, dass die Krümmung während des Einsetzens der Platte (1, 13) elastisch geradegerichtet wird, so dass die Platte (1, 13) dazu neigt, ihre anfängliche gekrümmte Form wiederzuerlangen, und wobei die Verankerungsmittel Verankerungsbolzen (7, 8, 9, 28; 14, 15, 16, 17, 18) sind, die fest an der Platte (1, 13) fixiert ausgebildet sind, so dass die Neigung der Platte (1, 13), ihre anfängliche gekrümmte Form nach ihrer Installation wiederzuerlangen, bewirkt, dass die Bolzen einen Druck auf die Wände ihrer Öffnungen in den Wirbeln ausüben.

2. Vorrichtung gemäß Anspruch 1, **dadurch gekennzeichnet, dass** die Platte (1, 13), wenn sie unbelastet ist, entlang ihrer Querrichtung (YY) eine Krümmung aufweist, die eine Konkavität der rückwärtigen Fläche (6) der Platte (1, 13) entlang ihrer Querrichtung (YY) erzeugt.

3. Vorrichtung gemäß einem der Ansprüche 1 bis 2, **dadurch gekennzeichnet, dass** die Verankerungsbolzen (7, 8, 9, 28; 14, 15, 16, 17, 18) einstückig mit der Platte (1, 13) ausgebildet sind.

4. Vorrichtung gemäß einem der Ansprüche 1 bis 3, **dadurch gekennzeichnet, dass** die seitlichen Ränder (4, 5) der Platte (1, 13) mindestens eine Konkavität aufweisen.

5. Vorrichtung gemäß Anspruch 3 oder 4, **dadurch gekennzeichnet, dass** die Verankerungsbolzen (7, 8, 9, 28; 14, 15, 16, 17, 18) Anti-Rückkehr-Kanten (12) oder Anti-Rückkehr-Widerhaken (35) aufweisen.

6. Vorrichtung gemäß einem der Ansprüche 3 bis 5, **dadurch gekennzeichnet, dass** die Platte (1) vorgesehen ist, um zwei Wirbel zu überspannen, und vier Verankerungsbolzen (7, 8, 9, 28) aufweist, von denen zwei (7, 9) vorgesehen sind, um in einen der Wirbel einzudringen, und die anderen beiden Bolzen (8, 28) vorgesehen sind, um in den anderen der Wirbel einzudringen.

7. Vorrichtung gemäß einem der Ansprüche 3 bis 5, **dadurch gekennzeichnet, dass** die Platte (13) vorgesehen ist, um drei Wirbel zu überspannen, und fünf Verankerungsbolzen (14, 15, 16, 17, 18) aufweist, von denen einer (18) vorgesehen ist, um in den mittleren Wirbel einzudringen.

8. Vorrichtung gemäß einem der Ansprüche 1 bis 7, **dadurch gekennzeichnet, dass** die Platte (1, 13) Vertiefungen oder Öffnungen (37) aufweist, in die ein zwischen den wirbeln platziertes und durch die Wirkung der Platte (1, 13) zusammengedrücktes Knochentransplantat eindringen kann.

## Revendications

1. Dispositif d'ostéosynthèse rachidienne consistant en une plaque (1, 13) munie de moyens d'ancrage et destinée à être fixée, par lesdits moyens d'ancrage, chevauchant au moins deux vertèbres d'un patient, où ladite plaque (1, 13) possède, lorsqu'elle est non contrainte, une courbure le long de ses deux directions longitudinale (XX) et transversale (YY), et où la plaque (1, 13) possède une élasticité qui permet à ladite courbure d'être redressée élastiquement durant la mise en place de la plaque (1, 13) de sorte que la plaque (1, 13) tende à retrouver sa forme courbée initiale, et où lesdits moyens d'ancrage sont des goujons d'ancrage (7, 8, 9, 28 ; 14, 15, 16, 17, 18) qu'on a fixés solidement à la plaque (1, 13) de sorte que la tendance de la plaque (1, 13) à retrouver sa forme courbée initiale après son installation force les goujons à exercer une poussée sur les parois de leurs orifices dans les vertèbres.

2. Dispositif selon la revendication 1, **caractérisé en ce que** la plaque (1, 13) possède, lorsqu'elle est non contrainte, une courbure le long de sa direction transversale (YY) qui produit une concavité de la surface arrière 6 de la plaque (1, 13) le long de sa direction transversale (YY).

3. Dispositif selon l'une des revendications 1 à 2, **caractérisé en ce que** lesdits goujons d'ancrage (7, 8, 9, 28 ; 14, 15, 16, 17, 18) sont faits d'un seul tenant avec la plaque (1, 13).

4. Dispositif selon l'une des revendications 1 à 3, **caractérisé en ce que** les bords latéraux (4, 5) de la plaque (1, 13) ont au moins une concavité.

5. Dispositif selon la revendication 3 ou 4, **caractérisé en ce que** les goujons d'ancrage (7, 8, 9, 28 ; 14, 15, 16, 17, 18) ont des nervures anti-retour (12) ou des barbes anti-retour (35).

6. Dispositif selon l'une des revendications 3 à 5, **caractérisé en ce que** la plaque (1) est destinée à chevaucher deux vertèbres et possède quatre goujons d'ancrage (7, 8, 9, 28), deux (7, 9) d'entre eux étant destinés à pénétrer dans l'une desdites vertèbres et les deux autres goujons (8, 28) étant destinés à pénétrer dans l'autre desdites vertèbres.

7. Dispositif selon l'une des revendications 3 à 5, **caractérisé en ce que** la plaque (13) est destinée à chevaucher trois vertèbres et possède cinq goujons d'ancrage (14, 15, 16, 17, 18), l'un (18) deux étant destiné à pénétrer dans la vertèbre centrale.

8. Dispositif selon l'une des revendications 1 à 7, **caractérisé en ce que** la plaque (1, 13) possède des dépressions ou des orifices (37) dans lesquels un greffon osseux placé entre les vertèbres et comprimé par l'action de la plaque (1, 13) peut pénétrer.
